# EUROPEAN PATENT APPLICATION

(11) **EP 2 088 528 A2**
(43) Date of publication of application: **12.08.2009**
(21) Application number: 09152204.5
(22) Date of filing: 05.02.2009
(51) Int. Cl.: G06F 19/00

(54) **Rater resource allocation systems and methods**

(30) Priority: 05.02.2008 US 25871
(71) Applicant: Medavante, Inc., Hamilton, NJ 08619 (US)
(72) Inventor: Schmidt, Joseph, Newtown, PA 18940 (US); Johnson, Livingston, Skillman, NJ 08558 (US); Masotti, Matthew Clifford, West Windsor, NJ 08550 (US); Sukovich, Patricia, Ringoes, NJ 08551 (US)
(74) Representative: Delumeau, François Guy

(57) **Abstract**

Methods of allocating raters to assessment visits of studies, and tangible computer readable media including software that is adapted to control a computer to implement methods of allocating raters to assessment visits of studies, are provided. Raters are allocated by retrieving (1502) blinding information that includes at least one blinding criterion for a visit of a study, retrieving (1500) rater information for at least one rater associated with the study, comparing (1504) the retrieved rater information and the retrieved blinding information to identify one or more raters meeting the at least one blinding criterion and enabling (1512) selection of the identified one or more raters for allocation to the visit of the study. A rater may then be allocated to the visit by receiving a selection for at least one of the identified raters and allocating the at least one rater to the visit responsive to the received selection.

## Description

### FIELD OF THE INVENTION

The present invention relates to clinical trial studies and, more particularly, to rater resource allocation systems and methods for allocating raters to assessment visits of clinical trial studies.

### BACKGROUND OF THE INVENTION

Clinical trial studies ("studies") are used in the pharmaceutical industry to assess the effectiveness of pharmaceuticals. In a typical study, a sponsor of the study (such as a pharmaceutical company) selects one or more investigators (such as physicians affiliated with hospitals and/or clinics and/or physicians in group or private practices) to identify subjects for the study from a pool of candidates (such as patients of hospitals, clinics, or physician practices) and to assess the identified subjects throughout the study. The investigators may utilize raters to identify and assess the subjects.

A study generally includes a number of assessment visits. The initial assessment visit may be a screening visit performed to identify subjects from the pool of candidates. For eligible subjects, subsequent assessment visits may be performed to obtain a baseline for the identified subjects and to assess the identified subjects' responses to the pharmaceutical or indication being studied. During assessment visits, the raters assess the candidates/subjects using one or more known rating scales ("scales"), such as the Hamilton Depression (HAM-D) and Hamilton Anxiety (HAM-A) scales.

There is an ever-present need to improve the quality of studies in order to improve the value of performing these studies.

### SUMMARY OF THE INVENTION

The present invention is embodied in methods and systems of allocating raters to assessment visits of studies and to computer readable media including software that is adapted to control a computer to implement methods of allocating raters to assessment visits of studies. Raters may be allocated by retrieving blinding information that includes at least one blinding criterion for a visit of a study, retrieving rater information for at least one rater associated with the study, comparing the retrieved rater information and the retrieved blinding information to identify one or more raters meeting the at least one blinding criterion and enabling selection of the identified one or more raters for allocation to the visit of the study. A rater may then be allocated to the visit by receiving a selection for at least one of the identified raters and allocating the at least one rater to the visit responsive to the received selection.

### BRIEF DESCRIPTION OF THE DRAWINGS

The invention is best understood from the following detailed description when read in connection with the accompanying drawings, with like elements having the same reference numerals. When a plurality of similar elements is present, a single reference numeral may be assigned to the plurality of similar elements with a small letter designation referring to specific elements. When referring to the elements collectively or to a non-specific one or more of the elements, the small letter designation may be dropped. Included in the drawings are the following figures:
FIG. 1 is a block diagram of a centralized rater system for conducting studies according to an exemplary embodiment of the present invention;
FIG. 2 is a block diagram of a system utilized in conducting studies according to an exemplary embodiment of the present invention;
FIG. 3 is a graphical user interface for entering and viewing study visit information according to an exemplary embodiment of the present invention;
FIG. 4 is a flow chart of a method for allocating raters to assessment visits of studies according to an exemplary embodiment of the present invention;
FIG. 5 is a flow chart of a method for receiving and entering study information for use in the method of FIG. 4 according to an exemplary embodiment of the present invention;
FIG. 6 is a chart showing a remote assessment schedule according to an exemplary embodiment of the present invention;
FIG. 7 is a chart showing blinding requirements which may be applied to study visits according to an exemplary embodiment of the present invention;
FIG. 8 is a graphical user interface for entering and viewing site personnel information according to an exemplary embodiment of the present invention;
FIG. 9 is a graphical user interface for entering and viewing rater information according to an exemplary embodiment of the present invention;
FIG. 10 is a flow chart of a method for scheduling raters for study visits for use in the method of FIG. 4 according to an exemplary embodiment of the present invention;
FIG. 11 is a flow chart of methods for scheduling raters, rooms and observers for study visits for use in the method of FIG. 4 according to an exemplary embodiment of the present invention;
FIG. 12 is a graphical user interface for entering search information and viewing search results according to an exemplary embodiment of the present invention;

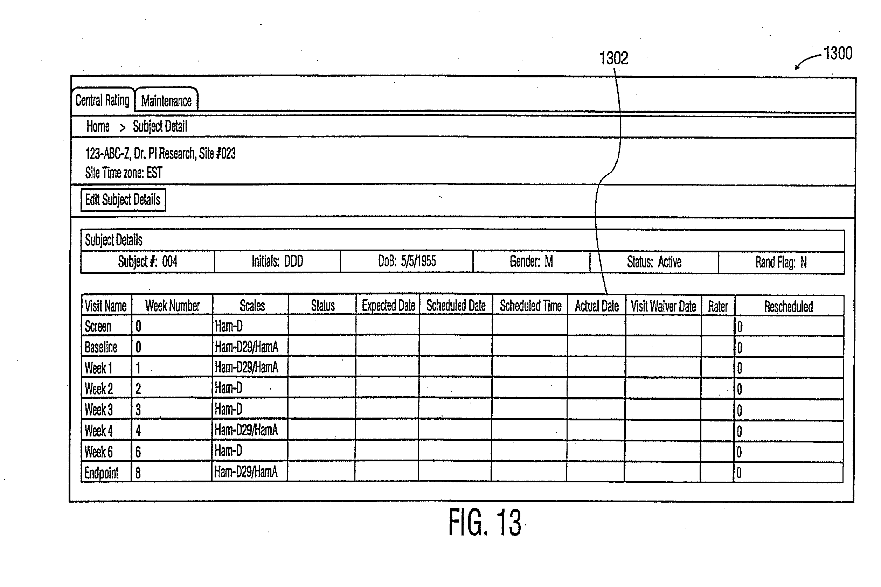

FIG. 13 is a graphical user interface for viewing and selecting visit information for a subject according to an exemplary embodiment of the present invention;
FIG. 14 is a graphical user interface for scheduling raters for visits of studies according to an exemplary embodiment of the present invention;
FIG. 15 is a flow chart of a method for enabling selection of raters who may be scheduled for a visit for use in the method of FIG. 4 according to an exemplary embodiment of the present invention;
FIG. 16 is a flow chart of a method for determining whether to enable selection of a rater for use in the method of FIG. 4 according to an exemplary embodiment of the present invention;
FIG. 17 is a graphical user interface showing a scheduled screening visit for a subject according to an exemplary embodiment of the present invention;
FIG. 18 is a graphical user interface showing all scheduled screening and baseline visits for a subject according to an exemplary embodiment of the present invention;
FIG. 19 is a flow chart showing a method for notifying appropriate parties that visits have been scheduled and for posting scheduled visits on appropriate parties' calendars for use in the method of FIG. 4 according to an exemplary embodiment of the present invention;
FIG. 20 is an exemplary notification used to notify investigators that visits have been scheduled according to an exemplary embodiment of the present invention;
FIG. 21 is an exemplary notification for notifying raters that they have been scheduled for a visit according to an exemplary embodiment of the present invention;
FIG. 22 is a graphical user interface for entering and viewing information according to an exemplary embodiment of the present invention.

### DETAILED DESCRIPTION OF THE INVENTION

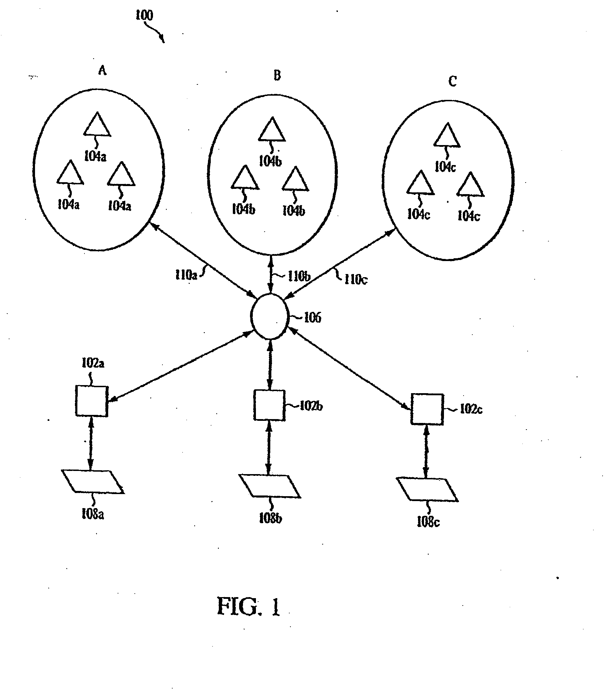

FIG. 1 is a block diagram of an exemplary centralized rater system 100 utilized in conducting studies from a central location ("central rater office") according to an embodiment of the present invention. System 100 includes, for example, one or more investigators 102, one or more candidates 104, one or more sponsors 108, and a central rater office 106. The central rater office 106 may include one or more facilities (not shown) remote to the investigators 102.

A sponsor 108 may authorize investigator(s) 102 to conduct a study for a new product (e.g., a new drug) or may conduct the study itself. The sponsor 108 or investigator 102 may authorize the central rater office 106 to provide raters to assess candidates in order to identify subjects for the study and to assess the identified subjects during the study, for example.

In the illustrated system 100, three different studies (represented by characters "A," "B," and "C") are being conducted. Each study may be initiated by a different sponsor 108a, 108b, and 108c, or multiple studies may be initiated by the same sponsor. For example, sponsor 108a may authorize investigator 102a to conduct study A. Likewise, sponsors 108b and 108c may authorize investigators 102b and 102c, respectively, to conduct studies B and C. In an alternative example, sponsor 108a may authorize two or more of investigators 102a-c to conduct two or more of the studies A-C.

In an exemplary embodiment, the central rater office 106 is authorized to perform an initial assessment visit to screen candidates in a pool of candidates associated with a study (e.g., candidates 104a associated with study A) to identify qualified subjects for the study and to perform subsequent assessments on the identified subjects. Raters located at the central rater office 106 may conduct assessment visits with candidates/subjects located at various sites (described below) associated with investigators 102 using remote communication media 110a. In an exemplary embodiment, raters at the central rater office 106 are trained such that the raters apply consistent assessment techniques for screening and assessing candidates/subjects.

Assessments may be conducted, for example, through audio and, optionally, video conferences between the raters located at the central rater office 106 (or, where applicable, at one of the central rater office's facilities) and respective candidates/subjects located at sites remote to the central rater office 106. The conferences may utilize communication media 110a such as telephone lines, local networks, global networks such as the Internet, and/or other communication medium that allow raters to remotely interact with the candidates/subjects. Data collected during the assessments is forwarded to the investigator 102 associated with the site where the subject was located during the assessment, who may then process and analyze the data or forward the data to the sponsor 108 of the study for processing and analysis. Alternatively, the data may be forwarded directly to the sponsor 108 for processing and analysis.

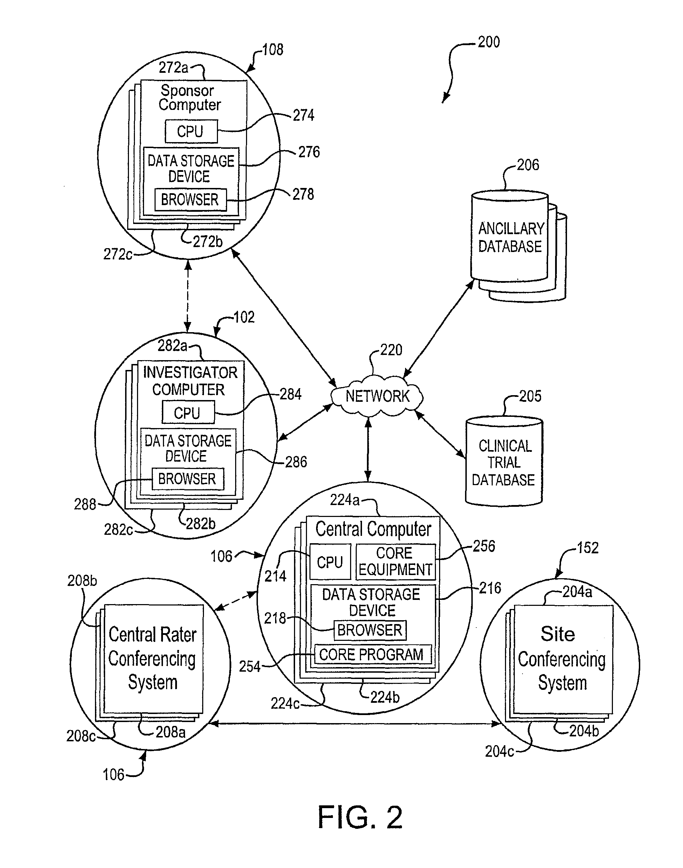

FIG. 2 depicts a block diagram of an exemplary system 200, which may be utilized in conducting a study according to an embodiment of the present invention. The illustrated system 200 includes one or more central computers (represented by computers 224a-c) located at central rater office 106 and/or at one or more of the central rater office's facilities; one or more central rater conferencing systems located at central rater office 106 and/or at one or more of the central rater office's facilities (represented by central rater conferencing systems 208a-c); one or more site conferencing systems (represented by site conferencing systems 204a-c) located at a physical site 152 associated with an investigator 102; one or more sponsor computers (represented by computers 272a-c) located at sponsor site 108; one or more investigator computers (represented by computers 282a-c) located at investigator site 102; a study database 205; and one or more ancillary databases 206.

A network 220 connects the various computers 224, 272, 282, and the databases 205, 206. The network 220 may broadly include, but is not limited to, any type of computer network or array of networks, or one or more wide area network, such as the Internet, intranet, satellite, and telephonic communication means. In addition, the network 220 may be a wireless network, and communication between computers may be through wireless connections, such as, for example, wireless Internet connections. Furthermore, network 220 may include other media of transmission such as, for example, a T-1 line.

Each of the central computers 224, the sponsor computers 272, and the investigator computers 282 includes, for example, one or more central processing units (CPUs) 214, 274, 284 and one or more data storage devices 216, 276, 286 comprising one or more browser programs 218, 278, 288, respectively, to allow access to, and communication through, the network 220. For example, in embodiments in which the network 220 is the Internet, the browser programs 218, 278, 288 may be, for example, Microsoft's Internet Explorer, or another Internet Browser. The data storage devices 216, 276, 286 may include various amounts of RAM for storing computer programs and other data. In addition, the central computers 224, sponsor computers 272, and investigator computers 282 may include other components typically found in computers, including one or more output devices such as monitors, other fixed or removable data storage devices such as hard disks, floppy disk drives and CD-ROM drives, and one or more input devices, such as mouse pointing devices, styluses, cameras, and keyboards. In addition, various other computer and computer related components may be utilized.

Generally, the central computers 224, the investigator computers 282, and the sponsor computers 272 may operate under and execute computer programs under the control of an operating system, such as Windows, Macintosh, UNIX, etc. Further, the computer programs may be tangibly embodied in a computer-readable medium, e.g., one or more data storage devices attached to a computer. Under the control of an operating system, computer programs may be loaded from data storage devices into computer RAM for subsequent execution by the CPU. The computer programs include instructions which, when read and executed by the computer, cause the computer to perform the steps necessary to execute elements of the present invention.

Central computers 224 may include core computer equipment 256, and data storage device 216 may include core program 254. The core computer equipment 256 and the core program 254 include all the equipment and programming necessary to support central rater site functions, including communication with the investigator and sponsor computers 282 and 272 as well as study coordination. Data compiled as a result of an assessment may ultimately be sent over the network from one party to one or more parties, as desired.

Central computers 224 may be located at (or accessibly from) the central rater office 106 and/or one or more of the central rater office's facilities. In an exemplary embodiment, central computers 224 may not be accessible to the raters so that true blinding of the raters may be carried out (i.e., the raters do not have access to stored assessment results).

Databases 205 and 206 may include, for example, any of a number of types of databases, including, for example, an Oracle® relational database system, commercially available from Oracle® corporation, a commercially available DB2 database, Microsoft Access, a Sybase® database, available from Sybase® Corporation, Microsoft® Structured Query Language (SQL) servers, or various Open DataBase Compliant (ODBC) databases.

The site conferencing system 204 and the central rater conferencing system 208 allow raters at the central rater office 106 and/or at one or more of the central rater office's facilities to conduct assessments of one or more candidates/subjects located at the site 152. In the exemplary embodiment shown in FIG. 2, the site conferencing system 204 and the central rater conferencing system 208 communicate directly with one another to establish and maintain a connection, independent of the central computer 224. By way of example, the site conferencing system 204 and the central rater conferencing system 208 may include equipment for teleconferencing, videoconferencing, connecting via the Internet, etc. In an exemplary embodiment, the site conferencing system 204 and the central rater conferencing system 208 include teleconferencing equipment such as the VSX5000 model provided by Polycom of Pleasanton, California, USA.

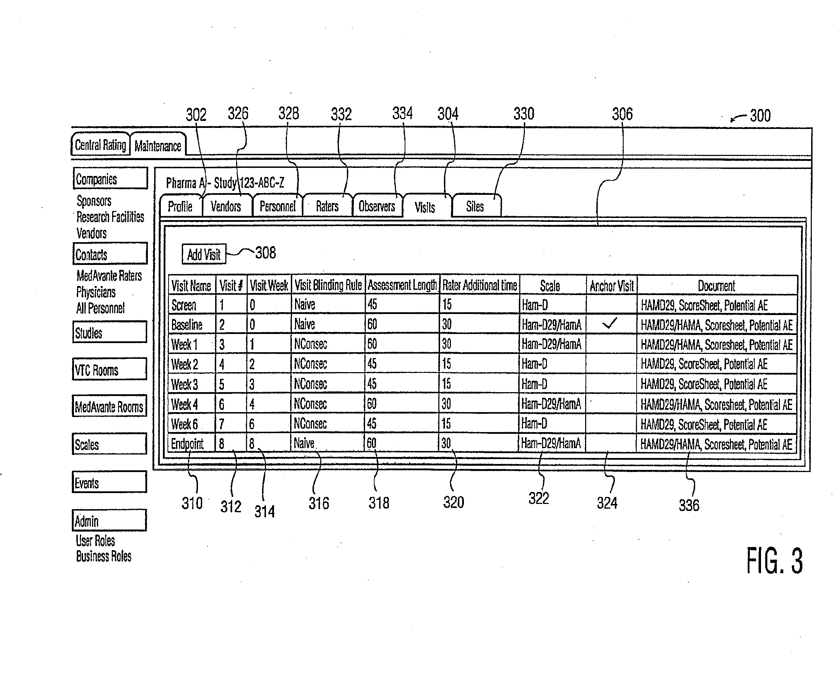

FIG. 3 is an exemplary graphical user interface ("GUI") 300 for entering and viewing information corresponding to an exemplary study. GUI 300 may be displayed on a monitor of the central computer 224. Data keyed via GUI 300 may be stored by the central computer 224 in the clinical trial database 205 and/or the ancillary database 206 or may be communicated to another computer. Additionally, other GUIs (such as described below) may be used to key, display, and/or communicate data.

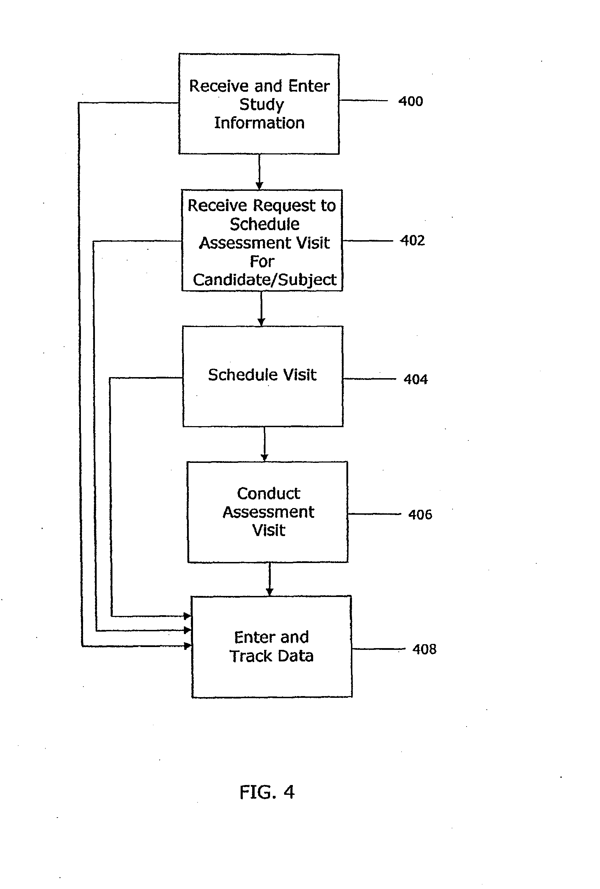

As an overview, FIG. 4 depicts a method including exemplary steps for allocating rater resources to study visits according to an embodiment of the present invention. At step 400, the rater allocation process begins when the central rater office receives and enters information for a study they have been authorized to perform. Receipt of the information may include receipt of pertinent information for conducting the study including, for example, a remote assessment schedule (described below), a name of the sponsor of the study, a name of a drug associated with the study and an anticipated number of subjects participating in the study. The received information and any other pertinent information may be entered ("keyed") into the central computer 224, as described in more detail below.

At step 402, the central rater office receives requests to schedule assessment visits for candidates/subjects. The requests may correspond with the remote assessment schedule provided at step 400. Each request may include, for example, an identifier of the site scheduling the visit, an identification ("ID") code for the subject (e.g., the subject's initials and/or unique ID number) to be assessed during the visit, a visit date and a visit time.

At step 404, the central rater office schedules the visit, e.g., based on a request from an investigator. Scheduling the visit at step 404 may include, for example, allocating an available rater to the visit who meets predetermined blinding and qualification requirements for the study, allocating required resources such as site and rater rooms, confirming the scheduled appointment with the investigator and notifying the rater of the scheduled appointment. Other steps may be carried out during scheduling, which are described below.

At step 406, the rater scheduled at step 404 (or potentially another rater if the original rater has been rescheduled) conducts the assessment for the scheduled visit. As described above, the rater may conduct an assessment with the candidate/subject over a remote communications medium. During the assessment, the rater is typically located in a rater interview room at the central rater office or at one of the central rater office's facilities, and the subject is typically located at a site associated with the investigator in a site interview room. To conduct the assessment, the rater administers one or more scales that are to be performed during that assessment visit as defined in the remote assessment schedule for the visit; an example of which is shown in xFIG. 6. In an exemplary embodiment, the rater administering the one or more scales is certified to administer the scales prior to conducting the assessment. The rater may administer the scales from a generated packet of information (not shown), which may include one or more scales to be administered, subject information, important site contacts and documents to be faxed to the proper parties, where required.

At step 408, data from the completed assessment is entered and tracked during the study or after the study has been completed. Once the rater is finished conducting the assessment, the rater may enter the data collected during the study into a central computer 224 or may provide the data to another person at the central rater site (a "user") for entry into the computer. Notifications and results of the assessment may then be sent to appropriate parties such as, for example, a safety contact at the site, a results contact for the sponsor, or to the investigator who may forward the information to the safety contact, the results contact, and/or other relevant parties.

Reports may be generated showing requested information such as, for example, assessment results or how many late appointments, rescheduled appointments, and cancelled appointments occurred during a particular study. Such information may be useful to the central rater office, for example, to project rater hiring needs for future studies as well as daily shift hours based upon trends of high and low activity.

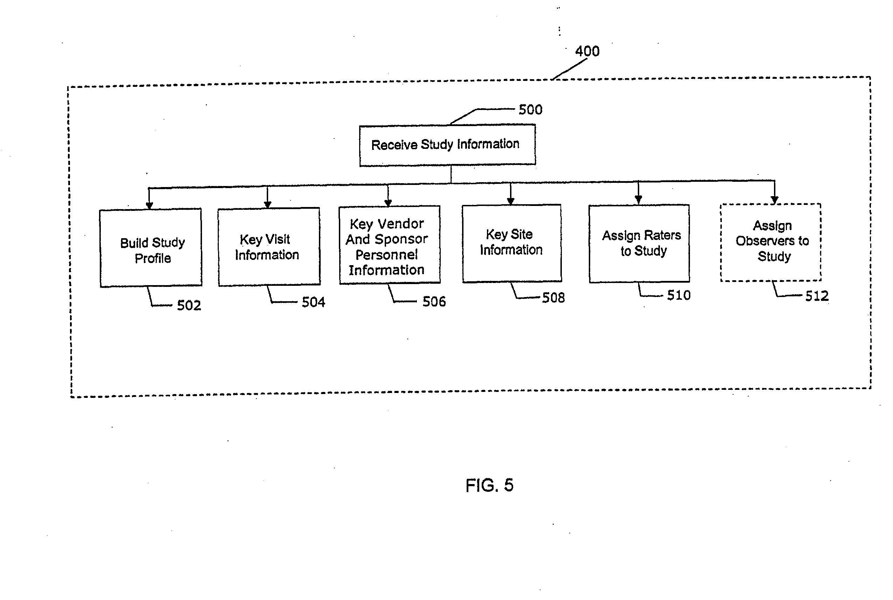

FIG. 5 is a flow chart of a method including exemplary steps for receiving and entering study information (step 400 of FIG. 4) according to an embodiment of the present invention. At step 500, the central rater office receives study information. In an exemplary embodiment, this information is received from the sponsor or the investigator. Such information may include, for example, the remote assessment schedule, the name of the sponsor, the name of the drug and/or indication being studied, a target number of sites, the anticipated number of subjects and anticipated first and last assessment visits. Additionally, this information may include information originating at the central rater office, e.g., study status.

At step 502, a study profile is built. The study profile may include general information corresponding to the study such as, for example, the name of the sponsor, a status of the study (e.g., whether the study is pending, active, etc.), the name of the drug and/or indication being studied, a study title, the target number of sites, the anticipated number of subjects, and the anticipated first and last visits. This information may be keyed at step 502 to build the study profile.

An acceptable visit delay may also be entered as part of the study profile. In an exemplary embodiment, the acceptable visit delay is an amount of time from the start time of a visit after which the sponsor or central rater office considers the assessment late; the acceptable visit delay may be different for each study. This information may be used when assessment results information is keyed, and may be particularly helpful in analysis of study data (described in more detail below).

In an exemplary embodiment, a GUI such as GUI 300 of FIG. 3 may be used to build a study profile, e.g., by selecting profile tab 302, which may bring up a new screen (not shown). The user may then key information (described above) in the new screen.

At step 504, visit information is entered. The visit information may include, for example, a visit name, a visit number, a visit week, one or more visit blinding rules, an assessment length, rater additional time, one or more scales, an anchor visit and documents related to each visit.

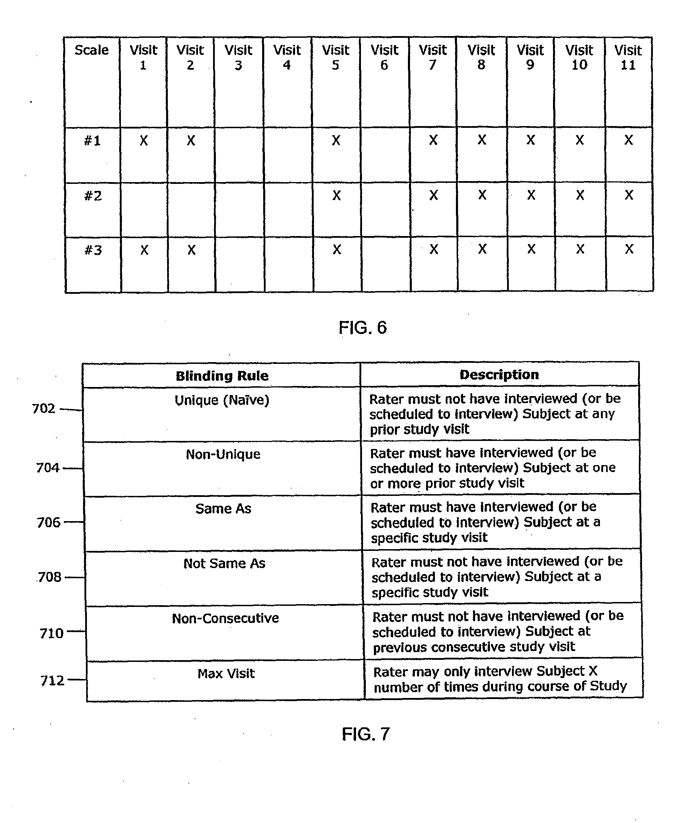

In an exemplary embodiment, the visit name (e.g., screening, baseline or general assessment), the visit number, and the one or more scales to be administered may be entered according to the remote assessment schedule for the study, an example of which is shown in FIG. 6. As shown in FIG. 6, the remote assessment schedule includes the visit numbers. The scales to be administered at each visit are marked by X's. Typically, visits #1 and #2 are the screening visit and the baseline visit, respectively. All remaining visits are typically general assessment visits. The week number may also be shown on the remote assessment schedule or it may be provided to the central rater office in a separate document.

Exemplary blinding criteria are depicted in the chart of FIG. 7. These blinding criteria, where included, are typically a part of the visit information. One or more blinding criterion for each visit may be selected by the central rater office, the sponsor, or by negotiation between the central rater office and the sponsor. Blinding criteria indicate an allowable level of interactivity between a rater and a subject to be scheduled. As shown in FIG. 7, a "unique" (or "naïve") blinding criterion 702 may be chosen for a visit if it is desirable that a rater has never interviewed the subject. A "non-unique" blinding criterion 704 may be chosen for a visit if it is desirable that a rater interviewed the subject at one or more prior visits. A "same as" blinding criterion 706 may be chosen for a visit if it is desirable that a rater interviewed the subject at a specific prior visit. A "not same as" blinding criterion 708 may be chosen for a visit if it is desirable that a rater has not interviewed the subject at a specific prior visit. A "non-consecutive" blinding criterion 710 may be chosen for a visit if it is desirable that a rater has not interviewed the subject at the previous visit. A "max visit" blinding criterion 712 may be chosen for the study if it is desirable that a rater only interview the subject a predetermined number of times over the course of the study.

The assessment length is the amount of time it typically takes for a rater to administer the scale or scales associated with the visit. The rater additional time is time required by the rater administering the assessment above what is needed to conduct the assessment for completing tasks associated with conducting the interview: for example, time needed to score the responses provided by the subject. The assessment length may be used to book rater remote assessment rooms or site assessment rooms (described below). The assessment length plus the rater additional time may be used to block out the proper amount of time for the rater to conduct the assessment when scheduling the rater for a visit (described below).

The scales that may be associated with a visit may include a series of questions which the rater may ask the subject. The questions may include, for example, questions about the subject's anxiety, tension, fears, ability to sleep, ability to concentrate, depression, pain, physical symptoms experienced since the subject's last visit, etc. The rater may then score the subject's answers according to the rater's training to generate raw data for the visit. The exemplary GUI 300 shows example scales that may be associated with a visit, e.g., HAM-D and HAM-A. One of skill in the art will recognize that other scales may be employed.

In an exemplary embodiment, a GUI such as GUI 300 of FIG. 3 may be used to enter visit information, e.g., by selecting visits tab 304 and entering information. When the visits tab 304 is selected, a window 306 is displayed. Window 306 displays information in the fields shown. For the example shown in FIG. 3, all visits for Study 123-ABC-Z have been entered. As shown, for each visit, the visit name is displayed in visit name field 310, the visit number is displayed in visit number field 312, the visit week is displayed in visit week field 314, the one or more visit blinding criterion is/are displayed in visit blinding rule field 316, the assessment length is displayed in assessment length field 318, the rater additional time is displayed in rater additional time field 320, the one or more scales is/are displayed in scale field 322, and a name(s) of or a link(s) to the one or more documents associated with the visit is/are displayed in document field 336. The baseline visit has been selected as the anchor visit for the study, as indicated by the check mark in anchor visit field 324 for the baseline visit. If window 306 is used to enter visit information, visit information may be entered by first selecting Add Visit icon 308. When the Add Visit icon 308 is selected, another window (not shown) is displayed in which information such as the visit name, visit number and visit week may be entered.

One or more blinding criterion may be selected for the visit by either keying the desired one or more criterion or by selecting the desired one or more criterion from, for example, a drop down menu. A scale(s) may be associated with the visit by keying the specific scale(s) to be administered or by selecting the specific scale(s) from, for example, a drop down menu. Assessment length field 318 may be automatically populated with a total amount of time preset for the scale(s) responsive to the selection of the scale(s). Additionally, names of documents or links to documents associated with the selected scale(s) may be automatically populated into document field 336. Later, the documents shown in the document field 336 may be included, potentially along with other pertinent information, in an automatically generated document, e.g., by the central computer 224 (see FIG. 2) which the rater may use to conduct the assessment. A visit may be selected as the anchor visit by checking a box indicating that the visit is the anchor visit, for example. The anchor visit may be used as a basis for generating projections in subsequent steps. It is also possible to conduct a study that does not use blinding criteria. In this case, when the user enters the visit information, the user may omit blinding criteria.

Referring back to FIG. 5, at step 506, vendor and sponsor personnel information may be entered. With respect to vendor personnel information, if outside vendors will be used in association with the study, the information may include contact information for pertinent personnel at the vendor. With respect to sponsor personnel information, the information may include contact information for pertinent personnel at the sponsor, such as, for example, a project manager or medical monitor. Contact information with respect to both vendor and sponsor personnel may include, for example, the person's study role (e.g., safety contact, notifications contact, etc.), the person's name, the company the person is associated with, the person's address, phone number, fax number and e-mail address and the mode by which information should be sent to the person (e.g., fax, e-mail, etc.).

In an exemplary embodiment, a GUI such as GUI 300 of FIG. 3 may be used to enter the vendor personnel information and the sponsor personnel information, e.g., by selecting vendors tab 326 or personnel tab 328, respectively. When the respective tab is selected, a new screen is displayed (not shown), through which the information may be keyed.

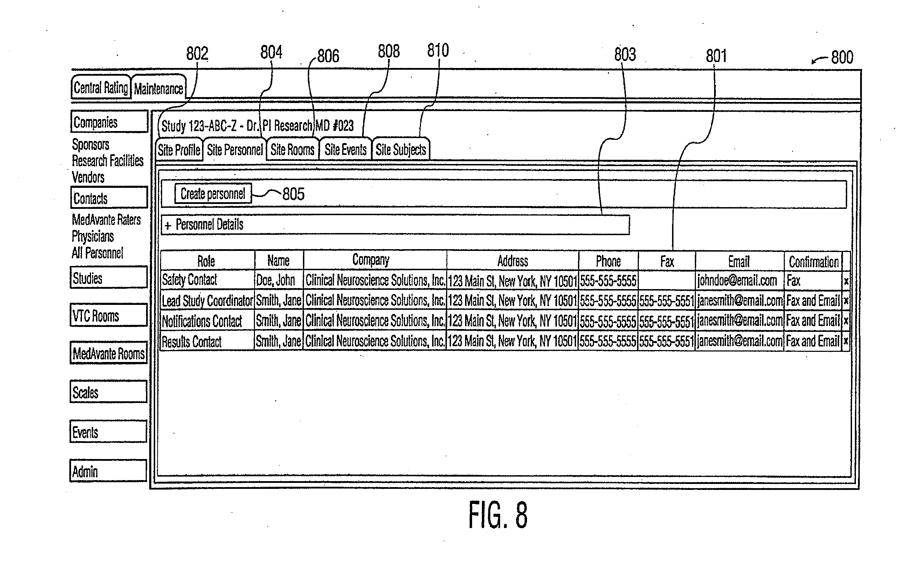

At step 508, site information may be entered. Site information may include, generally, site profile information, site personnel information, site room information, site events information and site subjects information. In an exemplary embodiment, a GUI such as GUI 300 of FIG. 3 may be used to enter sites to be associated with the study, e.g., by selecting sites tab 330. When sites tab 330 is selected, a new screen is displayed (not shown), through which each site may be keyed. To access site information that has already been entered, links for the entered sites may be selected upon selecting the sites tab 330. Selecting a link may bring the user to exemplary GUI 800 shown in FIG. 8, which displays site profile information.

Site profile information may include, for example, general information relating to the site such as, for example, the name and address of the site. In an exemplary embodiment, a GUI such as GUI 800 depicted in FIG. 8 may be used to enter profile information, e.g., by selecting site profile tab 802. When site profile tab 802 is selected, a new screen appears (not shown), through which site profile information may be keyed similar to the way the study profile information is keyed (described above).

Site personnel information may include, for example, site contacts such as the safety contact, a lead study coordinator, a notifications contact, and the results contact. Additionally, the site personnel information may include information associated with each contact such as role, name, company, address, phone number, fax number, e-mail address and by which mode the person would like to receive confirmations (e.g., by e-mail, fax, both e-mail and fax, etc.).

In an exemplary embodiment, a GUI such as GUI 800 may be used to enter personnel information, e.g., by selecting Create Personnel icon 805. When the Create Personnel icon 805 is selected, a new screen (not shown) appears, through which information, described above, may be keyed. In exemplary GUI 800, a window 801 shows information for site personnel that has already been entered. Window 801 is a drop down window, which is shown as open in FIG. 8. To close window 801, the plus sign next to "Personnel Details" in window 803 may be selected.

Site room information may include, for example, one or more rooms at the site's facility that have been set up with a conferencing system 204 to connect to the central rater office or facility with which remote assessments will be conducted via video or teleconference. In subsequent visit scheduling steps, specific site rooms entered at step 508 may be scheduled for the visit.

In an exemplary embodiment, a GUI such as GUI 800 of FIG. 8 may be used to enter site room information, e.g., by selecting site rooms tab 806. When site rooms tab 806 is selected, a new screen (not shown) is displayed, through which rooms at the site's facility that have been, for example, set up to connect to the central rating office via teleconference, may be entered by either keying the information or selecting the information, e.g., from a drop down menu.

Site events information may include, for example, information relating to site-specific events other than assessment visits that may be scheduled to take place with respect to the site. For example, it may be desirable to have raters from the central rater office train raters at the site, so that the site's own raters, in addition to the central rater office raters, may interview subjects participating in the study without deviating from the uniform training scheme initiated by the investigator.

In an exemplary embodiment, a GUI such as GUI 800 may be used to enter site events information, e.g., by selecting site events tab 808. When Site events tab 808 is selected, a new screen is displayed (not shown), through which site events information may be keyed.

Site subjects information may include, for example, information relating to subjects participating in the study such as, for example, the subject's identification number, the subject's initials and the subject's status with respect to the study (e.g., active, withdrawn, etc.). In an exemplary embodiment, a GUI such GUI 800 may be used to enter site subjects information, e.g., by selecting site subjects tab 810. When site subjects tab 810 is selected, a new screen (not shown) is displayed, through which the information may be keyed.

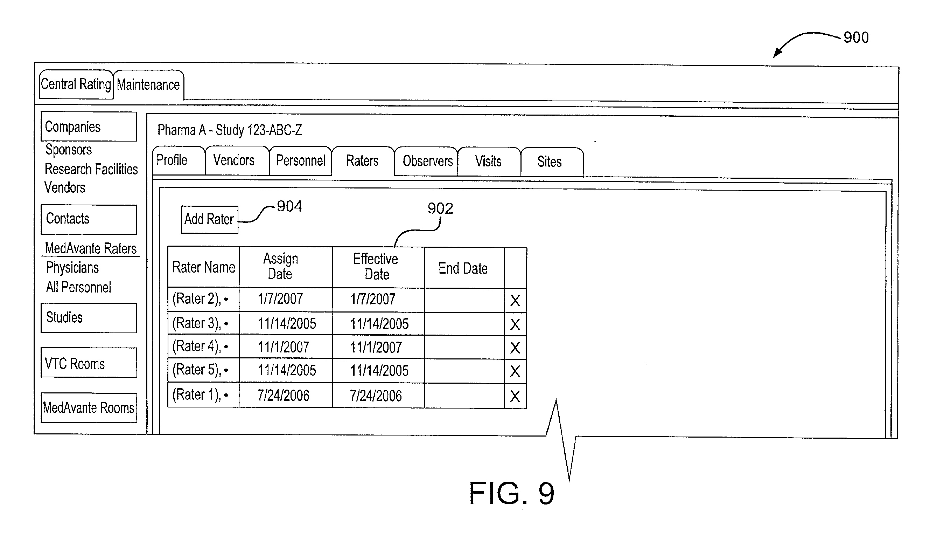

Referring back to FIG. 5, at step 510, raters may be assigned to the study. Generally, raters may be assigned to the study by selecting the study and then either keying in the rater or selecting the rater from, for example, a drop down menu. In an exemplary embodiment, a GUI such as GUI 300 may be used to assign raters to a study, e.g., by selecting raters tab 332. Selecting raters tab 332 may bring the user to exemplary GUI 900 shown in FIG. 9. As shown, raters already assigned to the study, along with their assignment and effective dates (described below), are displayed in window 902. To assign a rater to the study, the user may select Add Rater icon 904 and then either key in the rater information or select the rater from, for example, a drop down menu.

During step 510, raters may also be assigned assignment dates (e.g., the day the rater was assigned to the study) and effective dates (e.g., the day the rater has completed training for the scale(s) to be administered during the study). Because the effective date depends on the rater completing training, the entering rater effective dates portion of this step may take place later in the process (e.g., when the rater has completed training). An end date may be entered with respect to a rater if the rater is no longer associated with the study (e.g., due to re-assignment, resignation, conflict, termination, etc.).

At step 512, observers may optionally be assigned to the study. This may be performed in a manner similar to the step of assigning raters to the study (described above). An observer may be assigned to a study if, for example, a rater is assigned to the study who must be observed for purposes of, for example, reviewing the rater's ongoing qualification. The central rater office or sponsor may, for example, assign different qualification levels to raters based on, for example, their ability to administer scales accurately in accordance with their training. The qualification levels may be scale specific. In one embodiment, an observer is a rater who has obtained a predetermined qualification level and may be scheduled for visits in order to observe the rater assigned to the visit. In other embodiments, observers may be other personnel separate from the raters.

In an exemplary embodiment, a GUI such as GUI 300 may be used to assign an observer to a study, e.g., by selecting observers tab 334. Selecting observers tab 334 causes a new screen (not shown) to be displayed, through which a user may key the names of the observers or select the observers from, for example, a drop down menu.

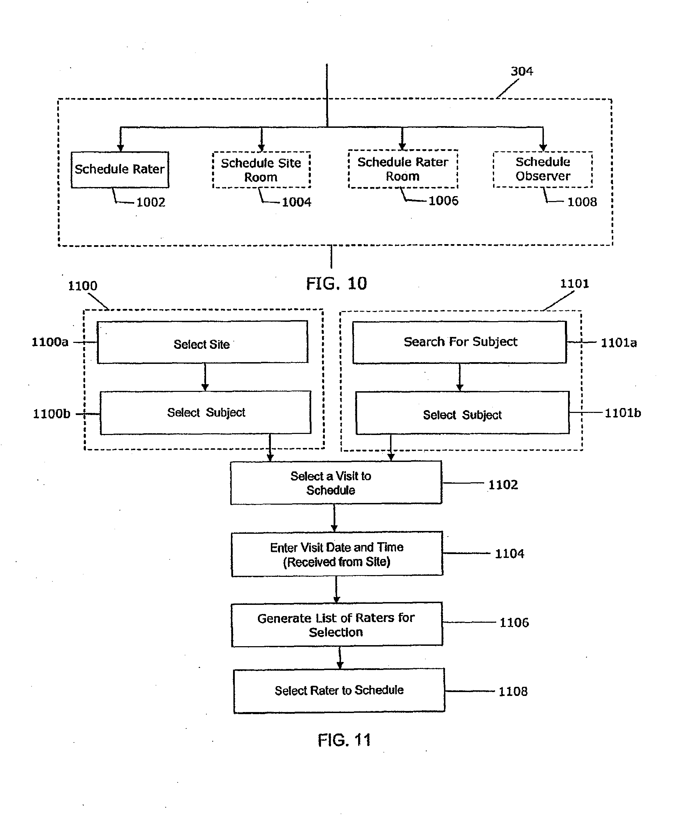

FIG. 10 is a flow chart of exemplary steps for scheduling visits (step 404 of FIG. 4). At step 1002, a rater is scheduled to conduct the assessment visit. At optional steps 1004, 1006 and 1008, a site room, a rater room, and/or an observer may be scheduled for the assessment visit, respectively.

FIG. 11 is a flow chart of exemplary steps for scheduling a rater for a visit (step 1002 of FIG. 10).

In an exemplary embodiment, at block 1100, the subject to be assessed during the visit is selected for scheduling. In accordance with this embodiment, the subject may be selected by first selecting a site at step 1100a (e.g., based on information obtained during step 402 of FIG. 4). Selection of the site results in a list of subjects associated with the selected site being displayed. The subject to be assessed may then be selected from the displayed list of subjects at step 1100b. In an exemplary embodiment, a GUI such as GUI 300 may be used to select the site, e.g., by selecting sites tab 330. When the user selects sites tab 330, a new screen is displayed (not shown) on which a list of entered sites may be displayed. The user may then select the site from the displayed list. When the user selects a site from the list, the exemplary GUI 800 of FIG. 8 is displayed. The user may then select the subject by first selecting site subjects tab 810 and then selecting the subject ID code or the subject's initials (e.g., based on information obtained during step 402 of FIG. 4) from a list displayed when site subjects tab 810 was selected (not shown).

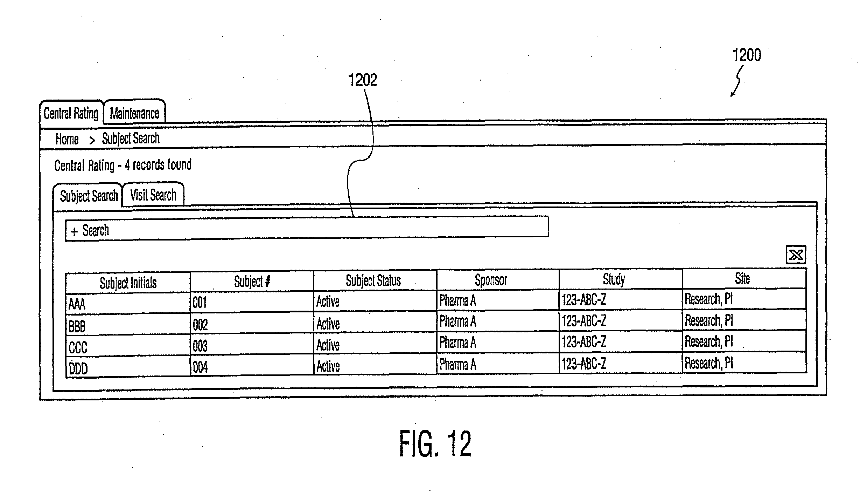

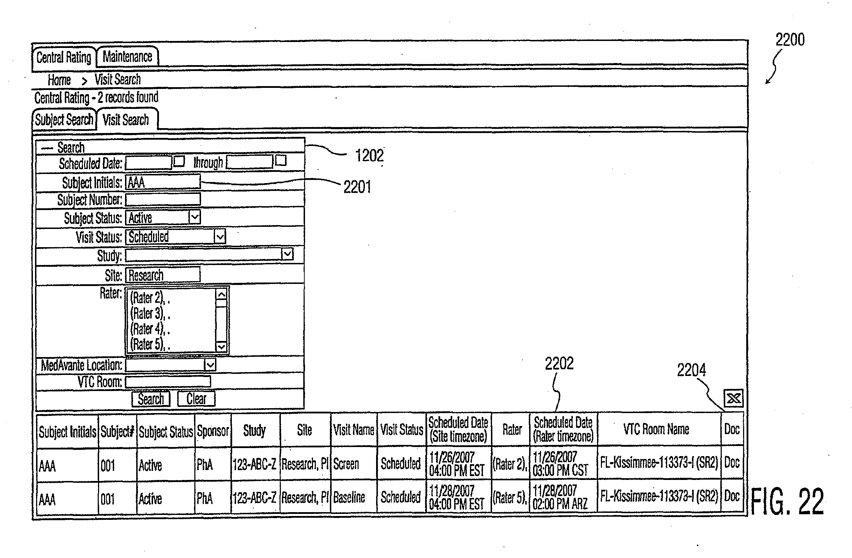

In an alternative embodiment, at block 1101, a user may select the subject to be assessed through a search. In accordance with this embodiment, the subject may be selected by first conducting a search for the subject at step 1101a. For example, the user may search a database 205/206 updated by the central computer 224 for the subject ID code or the subject's initials obtained during step 402 of FIG. 4. Search results may be displayed in a list including one or more subjects. The subject to be assessed may then be selected from the displayed list of one or more subjects at step 1101b. In an exemplary embodiment, a GUI such as GUI 1200 of FIG. 12 may be used to perform the search. In FIG. 12, the search function is implemented as a drop down menu 1202. The drop down menu 1202 is shown as closed in FIG. 12. FIG. 22 shows the drop down menu 1202 as open. In GUI 2200 of FIG. 22, the user has entered subject initials "AAA" in subject initials search field 2201. The results of the search entered using GUI 2200 are displayed for selection in window 2202.

At step 1102, the user selects the visit to be scheduled, e.g., from a GUI displaying a list of visits corresponding to the selected subject based on information obtained during step 402 of FIG. 4. For example, the user may select the visit from the list displayed in window 2202 of GUI 2200 or window 1302 of GUI 1300.

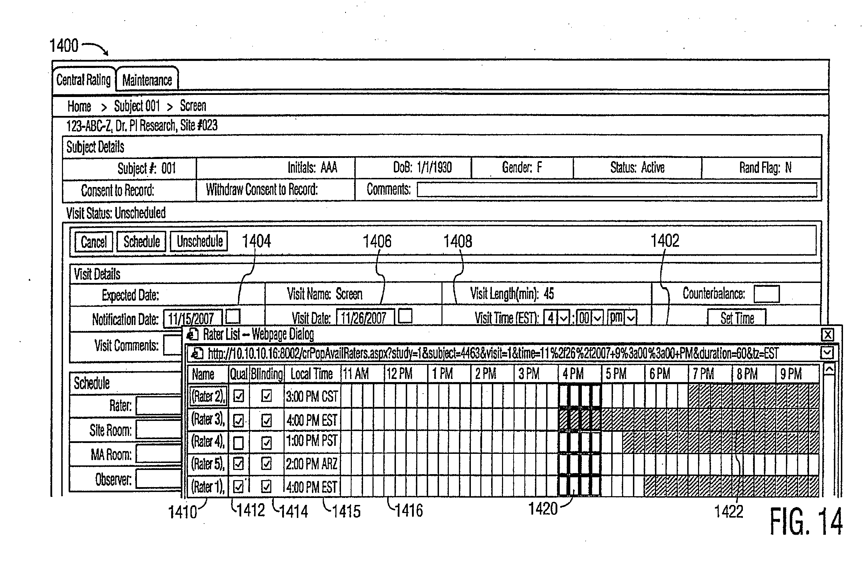

At step 1104, the user may enter the visit date and time (and optionally the notification date for data tracking purposes). FIG. 14 shows an exemplary GUI 1400 which may be used to enter the visit date, time and notification date according to an exemplary embodiment of the present invention. In particular, GUI 1400 depicts exemplary scheduling fields, which are partially obscured by a "Rater List -- Webpage Dialog" popup screen 1402 (described below). The scheduling fields may include, for example, notification date field 1404, visit date field 1406 and visit time field 1408. In an exemplary embodiment, a GUI such as GUI 1400 of FIG. 14 may be used to enter the visit date, the visit time and the notification date, for example, in fields 1404, 1406 and 1408, respectively.

In FIG. 14, the visit time is shown in EST (Eastern Standard Time) format. The visit time, however, may alternatively be shown in a different time zone, e.g., based on a selection by a user. In one embodiment, the visit time may automatically adjust to depict the time provided by the site in the local time of the central rater office, e.g., based on the difference in time of the site's time zone and the time zone where the central rater office is located. Accordingly, the user may simply enter the visit time supplied by the site without the need to convert the visit time to the local time.

At step 1106, a list of raters is generated for selection. The list of raters may automatically be generated after the user has entered the visit date and time at step 1104. In an exemplary embodiment, the list of raters may be displayed in a window such as a GUI (not shown) or a popup window such as popup window 1402 shown in FIG. 14. In an exemplary embodiment, only a subset of the listed raters are enabled for selection, which is described in further detail below. In the GUI 1400, raters 1, 2, 3, and 5 are enabled for selection (as indicated by their dark appearance) and Rater 4 is not enabled for selection (as indicated by its light appearance).

At step 1108, the user selects a rater to schedule. In an exemplary embodiment, the user selects a rater from the enabled raters in the list generated at step 1106 to schedule for the visit.

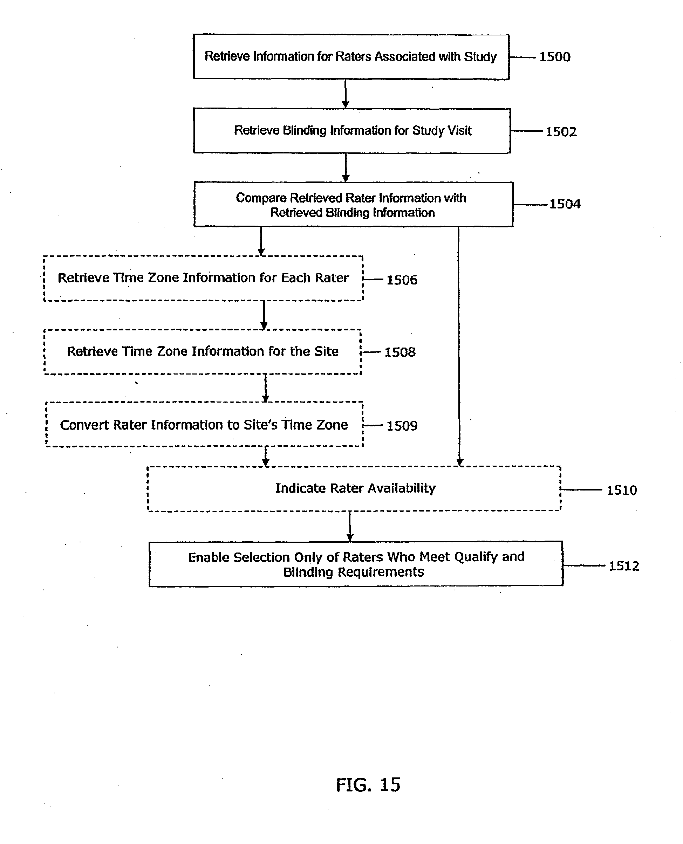

FIG. 15 is a flow chart of exemplary steps for enabling the selection of raters (step 1106 of FIG. 11). At step 1500, information for raters associated with the study is retrieved. Such information may include, for example, names of associated raters, whether each associated rater has been assigned an effective date (whether each associated rater has completed training) and/or when, if at all, each associated rater has previously assessed the subject.

At step 1502, blinding information for the study visit is retrieved. In an exemplary embodiment, the blinding information retrieved is the information entered during step 504 of FIG. 5. If no blinding information was entered during step 504 of FIG. 5, a null value may be retrieved during step 1502.

At step 1504, the retrieved rater information is compared with the retrieved blinding information. For example, information including when, if at all, each associated rater has previously interviewed the subject may be compared to the one or more blinding criterion set for the visit at step 504 of FIG. 5 to determine whether each rater is eligible to be scheduled for the visit based on the one or more blinding criterion. If no blinding information was entered during step 504 of FIG. 5, the retrieved information pertaining to whether, if at all, each rater has previously assessed the subject, is compared to the retrieved null value.

In an exemplary embodiment, the software may proceed directly from step 1504 to step 1510. In this exemplary embodiment, step 1510 includes retrieving rater availability information (e.g., the rater's schedule for the visit time and, optionally, for other time on the visit date) and indicating the rater availability information. The rater's availability may be indicated in the rater's time zone or in a time zone set as a default time zone in the software (e.g., the time zone in which the central rating office is located).

Steps 1506, 1508, and 1509 are an optional sequence of exemplary steps which may be carried out between steps 1504 and 1510 to indicate rater availability to the user in the site's time zone (or other time zone). At step 1506, time zone information for each rater is retrieved. At step 1508, time zone information for the site is retrieved (e.g., based on the central rater office or facility at which the rater is located). At step 1509, using the information retrieved at steps 1506 and 1508, the availability information is converted from the rater's time zone into the site's time zone. In this exemplary embodiment, step 1510 may further include indicating rater availability information in the site's time zone.

At step 1512, raters are enabled for selection. In an exemplary embodiment, raters are enabled for selection if they meet predetermined blinding and qualify rules for the study visit. If no blinding information was entered during step 504 of FIG. 5, depending on the configuration of the system, either all raters, all available raters, all raters meeting the training requirements or all available raters meeting the training requirements are enabled for selection during step 1512.

In an exemplary embodiment, a GUI such as GUI 1400 of FIG. 14 may be used to indicate rater availability in step 1510 and to enable selection only of raters who meet the training requirement(s) and/or the one or more blinding criterion in step 1512 of FIG. 15. As shown in popup window 1402 in FIG. 14, associated raters 2, 3, 4, 5 and 1 are listed sequentially on the left hand side of the window in name field 1410. Next to name field 1410 is qualify field 1412 and next to qualify field 1412 is blinding field 1414. For raters 2, 3, 5 and 1, a box in qualify field 1412 is checked and for raters 2, 3, 5, 4 and 1, a box in blinding field 1414 is checked. This indicates that raters 2, 3, 5 and 1 meet the training requirement(s) (i.e., they have been assigned an effective date that is on or before the scheduled visit date) and raters 2, 3, 5, 4 and 1 meet the one or more blinding criterion (as determined at step 1504). Because rater 4 does not meet the training requirement(s), rater 4 is not enabled for selection. On the other hand, because raters 2, 3, 5 and 1 meet both the one or more blinding criterion and the training requirement(s), they have been enabled to for selection. If no blinding information was entered during step 504 of FIG. 5, the box in blinding field 1414 may be checked for all displayed raters.

Next to the blinding field 1414 in FIG. 14 is local time field 1415. Local time field 1415 indicates the begin time for the visit and, in this exemplary embodiment, the begin time is displayed for each rater in the rater's local time zone. The begin time may, however, be displayed in the site's time zone or in the standard time zone set for the software, as described above.

Next to time field 1415 in FIG. 14 is a series of time blocks 1416 ranging from 11 AM to 9 PM (representing a range of time before and after the requested schedule visit time), where each time block 1416 represents 15 minutes. The series of time blocks 1416 may be altered based on results from the indicating rater availability step 1510 (described above) to provide a visual indication of rater availability. Here, time blocks (such as time block 1420) between 4 PM and 5 PM are outlined in gray to indicate the begin time in the site's time zone plus the time required to execute the scales and the additional rater time. Darkened blocks of time (such as time block 1422) indicate times during which the corresponding rater is unavailable. In one exemplary embodiment, unavailable time, such as, for example, time a rater is unavailable due to another scheduled visit, may be indicated in different ways. The different ways unavailable time may be indicated include, by way of non-limiting example, using different colors, different shapes, outline versus solid and different patterns.

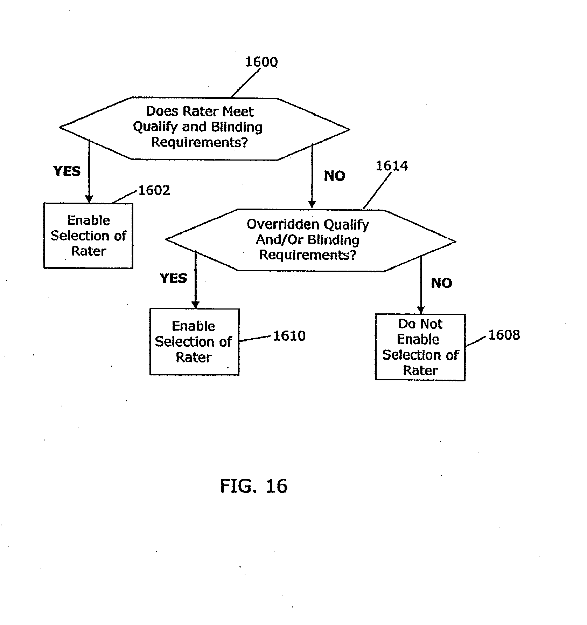

FIG. 16 is a flow chart of exemplary steps for enabling selection of raters (step 1512 of FIG. 15). In decision block 1600, a decision is made as to whether the rater meets the one or more blinding criterion and the training requirement(s). If the rater meets both, or if no blinding criteria have been set for the study, the rater is enabled for selection at step 1602. In decision block 1614, a decision is made as to whether the one or more blinding criterion and/or the training requirement(s) not met by the rater is/are overridden. Thus, raters that do not meet both the one or more criterion and/or requirement(s) may still be selected if the one or more blinding criterion and/or training requirement(s) is/are overridden, e.g., by a manager. If the one or more blinding criterion and/or the training requirements not met is/are overridden, processing proceeds at block 1610 with the rater being enabled for selection. If not, processing proceeds at block 1608 with the rater not being enabled for selection.

The override function described above may be useful in situations where few, if any, raters meet all criteria/requirements, but a rater must be scheduled for the visit. In an exemplary embodiment, only the one or more blinding criterion may be overridden. In an alternative exemplary embodiment, both the one or more blinding criterion and the training requirement(s) may be overridden.

In an exemplary embodiment, a user may enter the rater unavailable time and provide a reason why the rater is unavailable such as "lunch time," a meeting or a conflicting visit for which the rater is scheduled. If a user moves a cursor over a block of time in GUI 1400, the reason may be displayed. Thus, the user may see what events raters currently have scheduled, for example, to determine if another rater may be substituted for a rater's current scheduled appointment (e.g., the other rater is indicated as being in the office but only eating lunch at that time) and/or if the rater's scheduled appointment can be changed, to "free up" one or more raters in the event of scheduling conflicts.

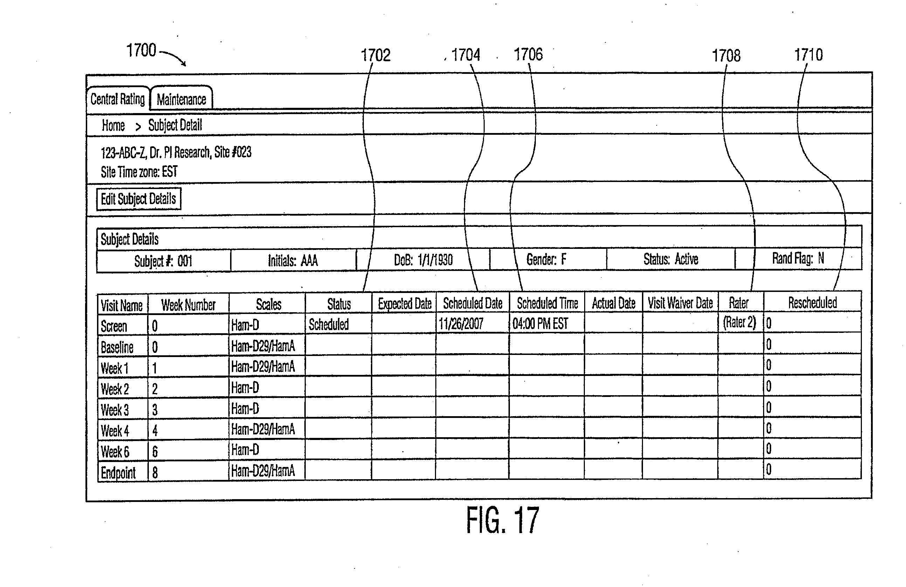

Once scheduling for the visit is complete, the visit is deemed scheduled. In an exemplary embodiment, a GUI such as GUI 1700 of FIG. 17 may be used to display whether a visit is scheduled. In GUI 1700, a status of the scheduled visit is shown in status field 1702, the date of the visit is shown in scheduled date field 1704, the time of the visit is shown in scheduled time field 1706 and the name of the scheduled rater is shown in rater field 1708. In illustrated GUI 1700, Rater 2 is scheduled for the screening visit for subject AAA to occur on November 26, 2007 at 4:00 EST. Because the scheduled visit has not been rescheduled, rescheduled field 1710 indicates that the visit has been rescheduled zero times. If the visit were to be rescheduled, a user may reschedule the appointment similar to the way in which the user scheduled the original appointment. When the visit is rescheduled, the number in the rescheduled field 1710 is incremented. The same steps may be performed for subsequent reschedules of the visit.

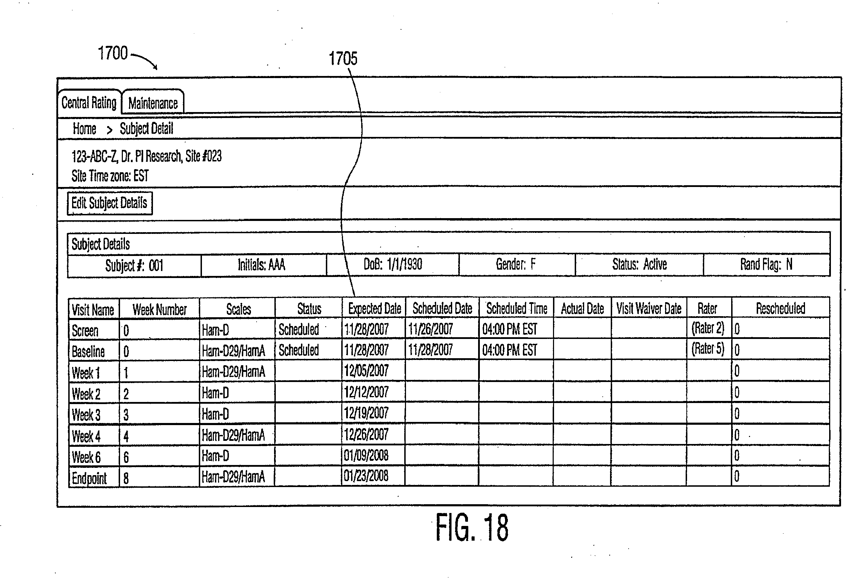

FIG. 18 shows the exemplary GUI 1700 of FIG. 17 with the baseline visit (also the anchor visit) for subject AAA also scheduled. Here, Rater 5 has been scheduled for subject AAA's baseline visit at 4:00 pm EST on November 28, 2007. Once the anchor visit is scheduled (i.e., in the illustrated embodiment), the expected date for the anchor visit and any prior and subsequent visits are displayed in expected date field 1705. Recall that in step 504 of FIG. 5, a visit week is assigned for each visit (e.g., week 0, week 1, week 2, etc.). If a prior or subsequent visit is assigned to the same week as the anchor visit, the expected date for that visit is displayed as the same date as the anchor visit. For all other visits, the expected date is displayed in increments of 7 days from the expected date for the anchor visit, depending on the week number assigned to the visit in step 504. For example, in FIG. 18, the baseline visit (the anchor visit) is scheduled to take place on November 28, 2007. The expected dates for both the baseline visit and the screening visit (both scheduled to take place in week 0) are displayed as November 28, 2007 (the date the anchor visit is scheduled to occur). The expected date for the week 1 visit is displayed as December 5, 2007 (7 days after the week 0 anchor visit expected date). The projected expected dates may allow the central rater office to plan by determining ahead of time how many raters may be needed on each given day as well as the times they may be needed. Further, they may allow the central rater office to remind investigators/sites about visits that are expected to occur in the near future.

Referring back to FIG. 10, while not required to successfully schedule the visit, at steps 1004, 1006 and 1008, a user may schedule a site room, a rater room and an observer for the visit, respectively. The steps for scheduling each of the site room, the rater room and the observer are similar to those described above with respect to scheduling the rater. To schedule a rater room or a site room, room availability information may first be retrieved. The user may then select an available room to schedule. As with scheduling raters, room busy time may be broken down into different categories, and the different time categories may be denoted differently. Further, if a user hovers the cursor over a block of time, information about what specific event is scheduled to take place in the room during the time block may be displayed. To schedule an observer, observer availability information may first be retrieved. The user may then select an available observer to schedule. As with scheduling raters and rooms, observer busy time may be broken down into different categories, and the different time categories may be denoted differently. Further, if a user hovers the cursor over a block of time, information about what specific event the rater has scheduled for that time may be shown.

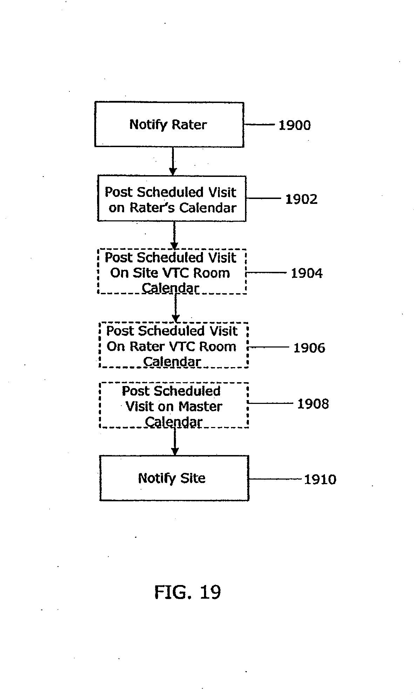

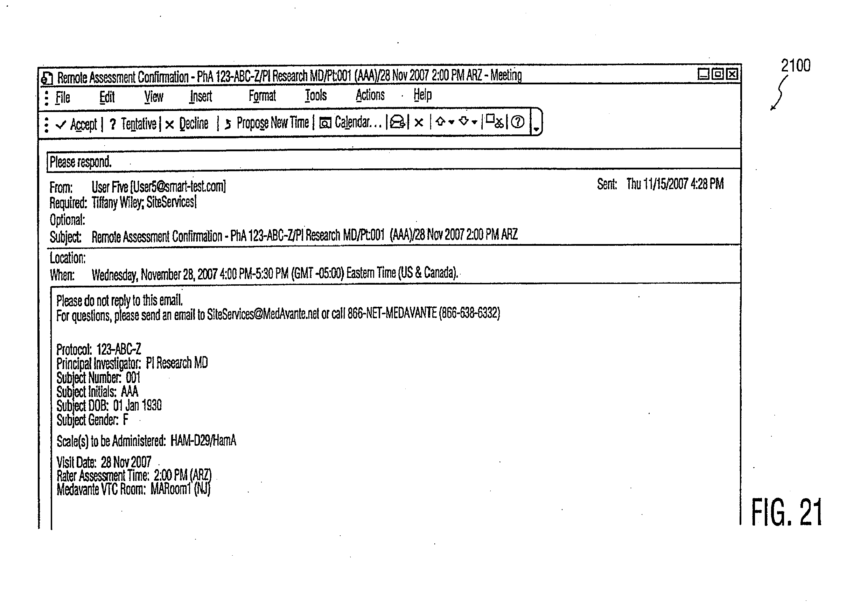

FIG. 19 depicts a flow chart of exemplary steps for notifying appropriate parties that the visit has been scheduled and for posting the scheduled visit on appropriate parties' calendars. At step 1900, the rater is notified of the scheduled appointment. In an exemplary embodiment, a meeting request is generated and e-mailed to the rater in order to notify the rater. Alternatively, the rater may be notified via e-mail or some other method (e.g., by facsimile or phone call). An exemplary appointment request 2100 is shown in FIG. 21. In an exemplary embodiment, information the rater needs regarding the scheduled visit may be included in the e-mail and may be, for example, automatically populated into the e-mail. Such information may include one or more of, for example, the study name ("protocol"), the name of the principal investigator, the subject's identification code, the subject's initials, the subject's date of birth, the subject's gender, the scale or scales to be administered, the visit date, the visit time and/or the rater room in which the assessment is scheduled to take place (if scheduled). If a meeting request or an e-mail is used to notify the rater of the scheduled appointment, the appointment request or e-mail may be automatically generated in response to the visit being scheduled.

At step 1902, the scheduled visit may be automatically posted to the rater's electronic calendar. If a meeting request was generated at step 1900, this may be done automatically in response to e-mailing the appointment request. If a rater room and/or a site room was scheduled at steps 1004 and 1006, the appointment may be posted to the site room calendar and the rater room calendar at steps 1904 and 1906, respectively. At step 1908, the appointment may be posted to a central rater office master calendar. Each of steps 1904, 1906 and 1908, if carried out, may be carried out responsive to the visit being scheduled.

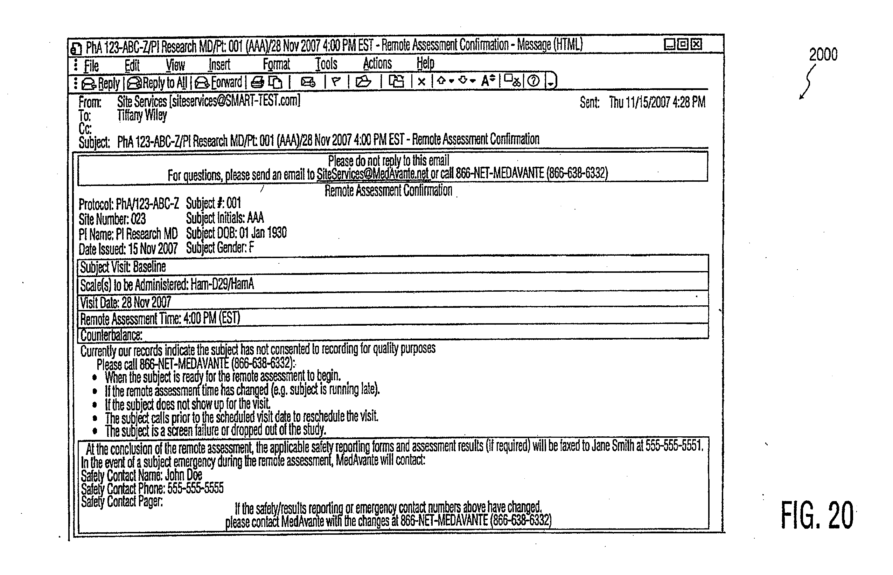

At step 1910, the site is notified that the appointment has been scheduled. The notifying may be done by e-mail, for example. FIG. 20 depicts an exemplary e-mail message 2000 for notifying the site. In an exemplary embodiment, information the site needs regarding the scheduled visit may be included in the e-mail and may be, for example, automatically populated into the e-mail. Such information may include one or more of, for example, the study name ("protocol"), the subject's identification code, a site number, the subject's initials, the name of the investigator, the subject's date of birth, a date issued, the subject's gender, the type of visit scheduled, the name(s) of the scale(s) scheduled to be administered, the visit date, the visit time, the central rater office's phone number, and contact information for the safety contact. The information may also include instructions including instructions on what to do if the subject cancels, reschedules, is a no show, or drops out of the study and/or instructions on what to do if the remote communications media fails. In an exemplary embodiment, the e-mail is automatically generated in response to the appointment being scheduled and the information is automatically populated into the e-mail message. In other embodiments, other means of notifying the site of the scheduled visit may be used (e.g., facsimile, phone call, etc.).

Referring back to FIG. 4, assuming the subject shows up for the scheduled visit, the scheduled rater conducts the assessment of the subject at step 406 by, for example, asking the questions associated with the scale(s) to be administered (as described above) and rating the subject's responses. The rater may then enter the data obtained from administering the scales or give the data to the user who may then enter the data. The entered data is stored in the clinical trial database 205 or the ancillary database 206. Additionally, at step 406, other information may be entered. For example, if the rater was late for the visit by more than the allowed amount of time, this information may be entered into the central computer 224. If the subject cancels, reschedules or drops out of the study, this information may be recorded and saved in the clinical trial database 205 and/or the ancillary database 206.

After the study information is received and entered during a study, at step 408, information relating to the study may be tracked and, in one embodiment, a report of the tracked information may be generated. For example, the study information may be searched (e.g., to retrieve a list of visits that have been rescheduled for a study) and the results incorporated into a report. In an exemplary embodiment, a GUI such as GUI 2200 shown in FIG. 22 may be used to search study information. As shown, one or more of the following fields may be searched: a scheduled date, a range of scheduled dates, a subject's initials, the subject's status with respect to the study, visits having a certain status, study name, site name, rater, investigator location or rater room.

Tracking data and generating reports at step 408 may be useful, as described above, for making business projections. For example, a report may include a number of late, rescheduled and cancelled visits for a study to help project a number of raters needed for future studies, on particular days and/or at particular times of day.

In addition to tracking information using the search screen shown in FIG. 22, searches performed, e.g., using GUI 2200 shown in FIG. 22, or other screens, may be used for other purposes. For example, GUI 2200 depicted in FIG. 22 shows a search conducted for scheduled visits for a specific active subject (AAA) from the "Research" site. Results window 2202, depicted at the bottom of FIG. 22, shows two retrieved visits matching the search results. On the far right of window 2202, a document field 2204 is shown. In field 2204, links are displayed that, when selected, cause the documents the rater needs to conduct the associated visit to be automatically generated. By way of another example, as described above with respect to step 1101, a user may search for a subject with respect to whom a site has requested a visit to be scheduled.

While the above embodiments describe allocating raters, observers and/or rooms to study visits, these embodiments may be modified to allocate resources in other applications. For example, the methods described with respect to FIGs. 5 and 11 may be modified for use in allocating trainers, candidates/subjects and/or actors (i.e., people who portray the symptoms of candidates/subjects) to training events for qualifying/certifying raters. Suitable adaptations of the above described embodiments for use in these other applications will be understood by one of skill in the art from the description herein and are considered within the scope of the present invention.

In accordance with these additional applications, information pertaining to a training program may first be entered. For example, a training profile may be built similar to the way a study profile is built in step 502 of FIG. 5. In this step, all information pertaining to the training program may be entered. Such information may include whether the training is for a particular scale and/or study, specific training events, event-specific score criteria and study outcomes (e.g., qualifications/certifications awarded to trainees upon successful completion of the program) may be entered during this step. Further, trainees/raters, trainers, candidates/subjects, and/or actors may be associated with the training program similar to the way raters, observers and rooms are associated with studies in steps 510 and 512 of FIG. 5 and steps 1004, 1006, and 1008 of FIG. 10.

To allocate trainers, patients and/or actors to training events, a trainee may be selected similar to the way a patient is selected in either step 1100 or 1101 of FIG. 11. A training event associated with the trainee/rater may then be selected for scheduling similar to the way a visit associated with the rater was selected in step 1102. A date and time for the training events may be entered similar to step 1104. A list of available trainers and/or candidates/subjects and/or actors may be generated similar to the way a list of available raters may be generated in step 1106. Similar to step 1108, a trainer, candidate/subject and/or actor may be selected to schedule for the training event.

Similar to the rater allocation methods, information pertaining to the training program may be tracked. For example, it may be desirable to track a trainee's progress in the training program by generating a report of completed and uncompleted training events for a specific trainee.

Although the invention is illustrated and described herein with reference to specific embodiments, the invention is not intended to be limited to the details shown. Rather, various modifications may be made in the details within the scope and range of equivalents of the claims and without departing from the invention.

## Claims

1. A method of allocating raters to assessment visits of studies, the method comprising:
retrieving (1502) blinding information that includes at least one blinding criterion for a visit of a study;
retrieving (1500) rater information for at least one rater associated with the study;
comparing (1504) the retrieved rater information and the retrieved blinding information to identify one or more raters meeting the at least one blinding criterion; and
enabling (1512) selection of the identified one or more raters for allocation to the visit of the study.

2. The method of claim 1, the method further comprising:
receiving a selection for at least one of the identified one or more raters; and
allocating the at least one rater to the visit responsive to the received selection.

3. The method of claim 1, the method further comprising:
receiving a request from a site to schedule the visit of the study for a subject, the request including at least a subject identifier, a visit date and a visit time.

4. The method of claim 3, the method further comprising:
retrieving availability information corresponding to at least the visit date for each of the one or more raters; and
displaying the availability information for each of the one or more raters meeting the at least one blinding criterion.

5. The method of claim 4, the method further comprising:
retrieving site time zone information corresponding to the site;
retrieving rater time zone information corresponding to the one or more raters;
converting the retrieved availability information corresponding to at least the visit date for each of the one or more raters into the site time zone using the retrieved site time zone information and rater time zone information,
wherein the step of displaying the availability information further comprises displaying the converted availability information corresponding to at least the visit date for each of the one or more raters.

6. The method of claim 3, the method further comprising:
retrieving availability information corresponding to at least the visit date for one or more conferencing rooms;
displaying the availability information for each of the one or more conferencing rooms;
receiving a selection for at least one of the displayed one or more conference rooms; and
allocating the at least one conference room to the visit responsive to the received selection.

7. The method of claim 1, wherein the blinding criterion are rules including one or more of:
(a) the rater may not be selected if the rater interviewed the subject at any prior study visit;
(b) the rater may not be selected if the rater did not interview the subject at one or more prior study visits;
(c) the rater may not be selected if the rater did not interview the subject at a predetermined study visit;
(d) the rater may not be selected if the rater interviewed the subject at the predetermined study visit;
(e) the rater may not be selected if the rater interviewed the subject at a previous consecutive study visit; or
(f) the rater may not be selected if the rater interviewed the subject at a number of previous study visits that is greater than or equal to a predetermined target number of total allowed study visits for the subject.

8. The method of claim 1, wherein the rater information includes each of the one or more raters' past visit information for the subject.

9. The method of claim 8, further comprising:
retrieving training requirements associated with the study;
retrieving rater training completion information;
comparing the retrieved training requirements with the retrieved rater training completion information; and
enabling selection of the identified one or more raters who also meet the training requirements as determined in the comparing step for allocation to the visit of the study.

10. The method of claim 9, further comprising:
enabling override of the blinding criterion, the training requirements, or both.

11. The method of claim 10, further comprising:
determining whether the blinding criterion, or the training requirements, or both, have been overridden for the rater; and
enabling selection of the rater if the blinding requirement, the training requirements, or both have been overridden.

12. The method of claim 1, further comprising:
retrieving site information;
retrieving subject information;
generating a document including the retrieved site information and the retrieved subject information.

13. The method of claim 12, further comprising:
retrieving scale information including questions corresponding to at least one scale associated with the visit,
wherein the generated document further includes the retrieved scale information.

14. The method of claim 12, wherein the generated document is selected from a group consisting of an e-mail or a facsimile.

15. A tangible computer readable medium including software that is adapted to control a computer to implement a method of allocating raters to assessment visits of studies, the processing method including:
retrieving blinding information that includes at least one blinding criterion for a visit of a study;
retrieving rater information for at least one rater associated with the study;
comparing the retrieved rater information and the retrieved blinding information to identify one or more raters meeting the at least one blinding criterion; and
enabling selection of the identified one or more raters for allocation to the visit of the study.

16. The tangible computer readable medium of claim 15, wherein the method implemented by the computer further includes:
retrieving availability information corresponding to at least a date of the visit to be scheduled for each of the one or more raters; and
displaying the availability information for each of the one or more raters meeting the at least one blinding criterion.

17. The tangible computer readable medium of claim 16, wherein the method implemented by the computer further includes:
retrieving site time zone information corresponding to the site;
retrieving rater time zone information corresponding to the one or more raters;
converting the retrieved availability information corresponding to at least the visit date for each of the one or more raters into the site time zone using the retrieved site time zone information and rater time zone information,
wherein the step of displaying the availability information further comprises displaying the converted availability information corresponding to at least the visit date for each of the one or more raters.

18. The tangible computer readable medium of claim 15,
wherein the blinding criterion are rules including one or more of:
(a) the rater may not be selected if the rater interviewed the subject at any prior study visit;
(b) the rater may not be selected if the rater did not interview the subject at one or more prior study visits;
(c) the rater may not be selected if the rater did not interview the subject at a predetermined study visit;
(d) the rater may not be selected if the rater interviewed the subject at the predetermined study visit;
(e) the rater may not be selected if the rater interviewed the subject at a previous consecutive study visit; or
(f) the rater may not be selected if the rater interviewed the subject at a number of previous study visits that is greater than or equal to a predetermined target number of total allowed study visits for the subject.

19. The tangible computer readable medium of claim 15, wherein the method implemented by the computer further includes:
retrieving the site information;
retrieving subject information; and
generating a document including the retrieved site information and the retrieved subject information.
